# EUROPÄISCHE PATENTSCHRIFT

(11) **EP 0 970 234 B1**
(45) Veröffentlichungstag und Bekanntmachung des Hinweises auf die Patenterteilung: **25.08.2004**
(21) Anmeldenummer: 98905197.4
(22) Anmeldetag: 09.03.1998
(51) Int. Cl.: C12P 7/06, A23K 1/00, C12M 1/107

(54) **VERFAHREN ZUR VERWERTUNG VON PFLANZLICHER BIOMASSE UND SCHNECKENPRESSE ZUR DURCHFÜHRUNG DIESES VERFAHRENS**
METHOD FOR USING A VEGETABLE BIOMASS AND A SCREW PRESS TO CARRY OUT SAID METHOD
PROCEDE DE VALORISATION D'UNE BIOMASSE VEGETALE ET PRESSE A VIS POUR LA MISE EN OEUVRE DE CE PROCEDE

(30) Priorität: 18.03.1997 CH 64797
(43) Veröffentlichungstag der Anmeldung: 12.01.2000
(73) Patentinhaber: 2B BIOTEC AG, 8600 Dübendorf (CH)
(72) Erfinder: HANSEN, Graeme, CH-8049 Zürich (CH); GRASS, Stefan, CH-8046 Zürich (CH)
(74) Vertreter: Patentanwälte Rehberg + Hüppe
(86) Internationale Anmeldenummer: PCT/CH1998/000094
(87) Internationale Veröffentlichungsnummer: WO 1998/041646

(56) Entgegenhaltungen:
- EP-A- 0 005 703
- EP-A- 0 213 023
- WO-A-82/01483
- DE-A- 2 919 518
- DE-A- 3 715 953
- DE-A- 4 423 099
- FR-A- 2 550 550
- US-A- 4 302 543
- US-A- 4 321 328
- US-A- 4 842 877
- PATENT ABSTRACTS OF JAPAN vol. 007, no. 086 (C-161), 9.April 1983 & JP 58 014995 A (MITSUBISHI JUKOGYO KK), 28.Januar 1983,

## Beschreibung

Die vorliegende Erfindung betrifft ein Verfahren zur Verwertung von Biomasse aus landwirtschaftlicher Ernte im wesentlichen im natürlichen Feuchtigkeitszustand, sowie eine Schneckenpresse zur Durchführung dieses Verfahrens.

Beispiele von in Betracht kommender Biomasse aus landwirtschaftlicher Ernte im wesentlichen im natürlichen Feuchtigkeitszustand sind Pflanzen oder Pflanzenteile mit besonders hohem Proteingehalt und/oder Zuckergehalt und deren Verarbeitungsderivate und Rückstände, insbesondere Klee, Luzerne und Gras, aber auch Bohnenpflanzen, Soja, Sorgho, Senf, Grünkohl, Futterrüben, Bananenblätter, Bagasse, Trester, Treber, Obstrückstände beispielsweise von Citrusfrüchten und Kernobst, und auch noch wuchernde Wasserpflanzen, beispielsweise Wasserlinse und Wasserhyazinthe, und dergleichen mehr.

Im Zusammenhang mit der Verwertung von Biomasse sind einzelne Verfahrenschritte wie Zerkleinerung, Verpressung, Hydrolyse, Kühlung, enzymatischer Abbau, Abbau durch Mikroorganismen, anaerobe Faulung, Gewinnung von Biogas, Abscheidung verschiedener Phasen wie im wesentlichen flüssiger, feststoffarmer, feststoffreicher bzw. gasförmiger Phasen usw. an sich bekannt, wie auch die Verwertung der einzelnen abgeschiedenen Phasen beispielsweise als Futtermittel, als Düngermittel, zur Ethanolgewinnung, zur Kohlendioxidgewinnung, als Brennstoff usw. Ebenfalls bekannt ist, in Schneckenförderern, die Bildung von abdichtenden Pfropfen aus dem behandelten Material zur Trennung verschiedener Kammerbereiche voneinander.

Dieser Stand der Technik ist jedoch nicht gesamthaft als einzelnes integriertes System bekannt. Im einzelnen:

WO-82/01483 offenbart ein Verfahren zur Verwertung eines Gemisches von organischem und anorganischem Material in städtischem Abfall sowie eine Vorrichtung zur kontinuierlichen Durchführung dieses Verfahrens. Das Material wird in zerkleinerter Form bereitgestellt; dann erfolgt eine sterilisierende Behandlung des gesamten Abfallmaterials mit Dampf im Überdruck; anschliessend erfolgt eine Entspannung, bei welcher das dampfbehandelte Abfallmaterial in eine fliessfähige, an organischen Stoffen reiche Phase und eine an anorganischen Feststoffen reiche Phase getrennt wird; danach erfolgt ein Abbau von organischen Stoffen der fliessfähigen Phase durch Einwirkung von Enzymen und/oder durch Fermentation mit Mikroorganismen, und die abbaubehandelte Phase wird in Feststoff und Flüssigkeit getrennt; die Flüssigkeit wird destilliert und der Rückstand wird erneut in Feststoff und Flüssigkeit getrennt; letztere Flüssigkeit wird anaerob fermentiert und die sich dabei entwickelnde Gasphase wird im Hinblick auf deren Verwertung abgeschieden; verwertet werden auch die Flüssigkeit aus der Fermentation und der Feststoff im Rückstand der Destillation.

Bei diesem Verfahren nach WO-82/01483 wird das Abfallmaterial nur einem Druck ausgesetzt, dabei jedoch nicht verdichtet (verpresst und kompaktiert), so dass es voluminös bleibt und die Feuchtigkeit beibehält.

Auch erfolgt die Trennung von organischen und anorganischen Inhaltsstoffen erst nach der Behandlung mit Druck.

Zudem erfolgt eine irreversible Expansion der Dampfphase, nach welcher die Energie des Dampfes nicht wiedergewonnen und zum Verfahren rückgeführt werden kann, weil sie im Kondenswasser verlorengeht.

Ausserdem erfolgt als allererster Schritt eine Sterilisierung des noch in voluminöser lockerer Form vorliegenden Guts, was bei Anwendung des Verfahrens auf städtischen Abfall sinnvoll ist, weil dieser Abfall die Umwelt gefährdet, bei Anwendung auf Biomasse aus landwirtschaftlicher Ernte jedoch nicht optimal ist, weil diese Biomasse die Umwelt nicht gefährdet und es nicht vordringlich ist, sie sofort im noch voluminösen Zustand zu sterilisieren. Die Sterilisierung kann jedoch beim Verfahren nach WO-82/01483 nicht auf einen späteren Schritt verschoben werden, denn sie erfolgt spontan zusammen mit der Behandlung mit Dampf im Überdruck als unerlässliche Vorstufe der Entspannung.

Generell ist das Verfahren nach WO-82/01483 darauf abgestimmt, vorerst die organischen und anorganischen Inhaltsstoffe voneinander zu trennen und erst danach deren Verwertung einzuleiten, während diese Trennung bei einer Biomasse aus landwirtschaftlicher Ernte gar nicht zweckmässig ist.

Das Verfahren nach WO-82/01483 vermag deshalb mit Biomasse aus landwirtschaftlicher Ernte im wesentlichen im natürlichen Feuchtigkeitszustand keine wirtschaftlich optimalen Ergebnisse zu ergeben.

DE-4423099 offenbart ein Verfahren zur Verwertung von Biomasse aus landwirtschaftlicher Ernte sowie eine Schneckenpresse zur Durchführung des Verfahrens. Die zerkleinerte Biomasse wird in einer Schneckenpresse verpresst und in eine feststoffarme und eine feststoffreiche Phase getrennt; die feststoffarme Phase wird abgeschieden; danach erfolgt eine nicht näher beschriebene Fermentation. Dieses Verfahren eignet sich zur Herstellung von Biogas, nicht jedoch zur Herstellung der wirtschaftlich höherwertigen Produkte Ethanol, Proteinkonzentrat und Fasern. Es erfolgt keine Dampfbehandlung, Expansion und Kühlung des Ausgangsmaterials.

US-4302543 offenbart ein Verfahren zur Verwertung von Biomasse aus landwirtschaftlicher Ernte. Die zerkleinerte Biomasse wird ohne vorgeschaltete Trennung von Feststoff und Flüssigkeit in einem Extruder unter Hochdruck und Hochtemperatur verpresst; nach hinreichender Kühlung wird das Gut durch Einwirkung von Enzymen hydrolysiert. Dieses Verfahren wurde zur Verarbeitung von stärkehaltigen Rohstoffen wie Mais, Weizen, Reis, Gerste, Roggen und Hafer entwickelt, es ermöglicht aber nicht die Verarbeitung von primär zellulosehaltigen Rohstoffen zu Ethanol, Proteinkonzentrat und Fasern. Zudem erfolgt keine Verpressung des Materials zur Trennung von Feststoff und Flüssigkeit und keine Rückgewinnung von Dampf nach erfolgter Hitzebehandlung. Eine Entspannung des unter Hochtemperatur und Hochdruck stehenden Guts wird in der Beschreibung als vorbekannt bezeichnet.

JP-58-014995 (wie in Patents Abstracts of Japan nachzulesen) offenbart die Verbesserung eines Fermentationsverfahrens durch vorgängige Beseitigung von nicht fermentierbarem Ballaststoff. Das Fermentationsgut wird in einer Schneckenpresse verpresst und dabei in zwei Phasen getrennt; die feststoffreiche Phase wird abgeschieden; dann werden die feststoffarme Phase fermentiert, das dabei entwickelte Biogas abgeschieden und verwertet, die verbleibende fliessfähige Gemischphase ihrerseits in zwei Phasen getrennt, und die beiden Phasen verwertet. Es erfolgt keine Dampfbehandlung, keine Kühlung und keine Zugabe von zur Herstellung von Ethanol und Nebenprodukten benötigten Enzymen und/oder Hefen.

DE-2919518 offenbart ein Verfahren zur Verwertung von Biomasse aus landwirtschaftlicher Ernte im wesentlichen im natürlichen Feuchtigkeitszustand sowie eine Schneckenpresse zur Durchführung des Verfahrens. Die zerkleinerte Biomasse wird mit Dampf im Überdruck behandelt; nach einer Kühlung erfolgt eine Fermentation mit Mikroorganismen ggf. unter Zugabe von Hefe und erst danach in der Schneckenpresse eine Trennung des Gemisches in zwei Phasen, welche verwertet werden. Dieses Verfahren wurde zur Gewinnung von Ethanol aus dem in Zuckerrohr enthaltenen freien Zucker entwickelt und eignet sich nicht zur Gewinnung von Ethanol aus Cellulose. Des weiteren werden keine Enzyme zur Spaltung der im Rohstoff enthaltenen Fasern eingesetzt.

US-4321328 offenbart ein Verfahren zur Rezyklierung von ethanolhaltiger Brühe, das bei der Gewinnung von Ethanol aus cellulosehaltigen Rohstoffen einsetzbar ist. Dabei wird der Rohstoff durch rezyklierte ethanolhaltige Brühe aufgeschlämmt und dann mechanisch zerfasert, durch Einwirkung von Enzymen verzuckert und mit Mikroorganismen fermentiert. Annahme, Konditionierung und Vorbehandlung des Rohstoffes werden nicht näher beschrieben.

EP-0005703 offenbart ein Verfahren zur Verwertung von festen Haushaltsabfällen und Biomasse. Im Falle der Verarbeitung von Haushaltsabfällen werden zunächst, in mehreren Schritten, verschiedene Inhaltstoffe voneinander getrennt. Anschliessend erfolgt bei allen Rohstoffen ein Zermahlen der hydrolysierbaren Inhaltstoffe, eine Sterilisierung und eine Verzuckerung mittels schwefliger Säure, eine Neutralisation, eine Fermentation, eine Trennung von Feststoff und Flüssigkeit sowie eine Destillation der flüssigen Phase.

EP-0213023 offenbart ein Verfahren zur Herstellung von Ethanol und Gluten aus Getreide.

US-4842877 offenbart ein chemisches Verfahren zur Verarbeitung von Biomasse aus landwirtschaftlicher Ernte durch Abbau der Holzfaserstoffe (Lignocellulose) zu Futterzusatz. Als erster Schritt des Verfahrens erfolgt eine alkalische Hydrolyse bei etwas erhöhter Temperatur. Die gewonnene flüssige Phase wird mit einem Chelatbildner versetzt. Unter anderen chemischen Verfahrensschritten erfolgt eine Oxidation mit Wasserstoffperoxid und Sauerstoff in einem Extruder.

FR-2550550 offenbart ein Verfahren zur Verwertung von Biomasse aus landwirtschaftlicher Ernte. Als erster Schritt des Verfahrens erfolgt eine Hydrolyse unter Hochtemperatur und Hochdruck; anschliessend erfolgt eine Entspannung, bei welcher das Material in eine flüssige Phase und eine Feststoffphase getrennt wird; aus der Feststoffphase wird das Lignin mit einem Lösungsmittel entfernt; danach erfolgt wiederum eine Hydrolyse unter Hochtemperatur und Hochdruck; nötigenfalls folgt eine Verzuckerung durch Einwirkung von Enzymen. Ziel dieses Verfahrens ist die Verflüssigung und Trennung von Hemicellulose, Cellulose und Lignin.

DE-3715953 offenbart ein Verfahren zur Verwertung von cellulosehaltiger Masse wie papierhaltiger Abfall, Stroh und Holzabfall sowie eine Schneckenpresse zur Durchführung des Verfahrens. Anschliessend an die Zerkleinerung der Masse erfolgt in der Schneckenpresse eine Aufschliessung und Sterilisierung durch Erhöhung der Temperatur und des Druckes und danach in einem geschlossenen System unter Abfuhr von Sauerstoff ein Nachreifungsprozess, dessen Produkt einem Weiterverarbeitungsprozess unterzogen wird. Es erfolgt keine BeHandlung des Rohstoffes mit Dampf.

Bei dem im vorstehenden genannten Stand der Technik ergibt sich keine naheliegende Kombination vorbekannter Techniken, welche zur Vermeidung der im vorstehenden erwähnten, beim Verfahren nach WO-82/01483 auftretenden Nachteile führen könnte.

Insbesondere wurde bei diesem Stand der Technik noch keine wirtschaftlich vertretbare Umsetzung der einzelnen Verfahrensschritte in einem optimal betriebenen integrierten System erreicht. Dafür können mehrere Gründe genannt werden:
- Wenn zucker- oder stärkehaltige Rohstoffe verarbeitet werden, sind die Rohstoffkosten im Vergleich zum Produktpreis für Ethanol in der Regel zu hoch.
- Wenn Methangas (Biogas) hergestellt wird, erlaubt der tiefe Produktpreis lediglich die Verarbeitung entsorgungspflichtiger Rohstoffe. Eine wirtschaftliche Nutzung von Rohstoffen aus landwirtschaftlicher Ernte ist hingegen nicht möglich.
- Wenn ausschliesslich Ethanol hergestellt wird, ist die Wertschöpfung zu gering im Verhältnis zur teuren Ausführung des technisch anspruchsvollen Verfahrens. Dies verstärkt den wirtschaftlichen Druck zur Errichtung von Anlagen mit einer sehr grossen Verarbeitungskapazität. Bei solchen Grossanlagen sind hohe Entwicklungskosten und zudem hohe Kosten für den Transport der Rohstoffe vorzusehen.

Aufgabe der Erfindung ist es demnach, ein Verfahren zur Verwertung von Biomasse aus landwirtschaftlicher Ernte im wesentlichen im natürlichen Feuchtigkeitszustand vorzuschlagen, bei welchem die Wirtschaftlichkeit des Umsatzes von Energie, Eingangsstoffen und Ausgangsstoffen gesamthaft optimierbar und zudem den mengen- und wertmässigen saisonalen Schwankungen dieser Umsatzkomponenten optimal anpassbar ist.

Aufgabe der Erfindung ist es insbesondere, bei einem Verfahren der genannten Art zu erreichen:
- die Nutzbarmachung verschiedener in der Biomasse enthaltener Inhaltsstoffe;
- die Verknüpfung von Anforderungen bezüglich der Rohstoffaufbereitung und Rohstofflagerung mit Anforderungen bezüglich der Nutzbarmachung der Inhaltsstoffe im Verfahren selbst;
- die optimale Weiterverarbeitung und Nutzung der abgeschiedenen im wesentlichen flüssigen bzw. festen Phasen; und
- die Optimierung der Energiebilanz nicht nur der einzelnen Verfahrensschritte, sondern des Verfahrens als Ganzes

Zur Lösung dieser Aufgabe ist ein Verfahren der eingangs bezeichneten Art erfindungsgemäss gekennzeichnet durch die im Anspruch 1 angegebene Kombination von aufeinanderfolgenden Verfahrensschritten.

Eine Schneckenpresse zur Durchführung des erfindungsgemässen Verfahrens ist gekennzeichnet durch die im Anspruch 9 definierten Merkmale.

Vorteilhafte Weiterbildungen und Verwendungen des Verfahrens sowie Ausbildungen der Schneckenpresse sind in den abhängigen Ansprüchen definiert.

Das erfindungsgemässe Verfahren eignet sich ganz besonders zur Anwendung auf eine Biomasse mit einem Feuchtigkeitsgehalt von etwa 65 bis etwa 90 Gew.-% und einem Proteingehalt von etwa 10 bis etwa 30 Gew.-% der Trockensubstanz.

Mit dem erfindungsgemässen Verfahren wird unter anderem erreicht:
- Die Verarbeitung proteinreicher Rohstoffe ermöglicht die Herstellung von Proteinkonzentrat und Hefe als Nebenprodukte, was die Wertschöpfung des Verfahrens als Ganzes erhöht und zu dessen Wirtschaftlichkeit entscheidend beiträgt. Dadurch können auch Anlagen mit relativ kleiner Verarbeitungkapazität wirtschaftlich betrieben werden, was die Nutzung von bestehender Infrastruktur ermöglicht, den energieaufwendigen Rohstofftransport in Grenzen hält und folglich die Kosten senkt. Andererseits ist der anlagentechnische Mehraufwand zur Gewinnung und Qualitätssicherung der Nebenprodukte im Vergleich zu den gesamten Anlagekosten gering.
- Die in Betracht gezogenen Rohstoffe sind über einen längeren Zeiträum hinweg verfügbar und können frisch verarbeitet werden, wodurch die Trocknungskosten und Lagerverluste sowie der Platzbedarf und die Kosten der Lagerkapazität wegfallen. Beispielsweise kann bei der Verarbeitung von Klee und Gras der Winterbetrieb sichergestellt werden, indem im Sommer zusätzlicher Rohstoff zur Anlage angeliefert und dort gepresst, und der zusätzliche Pressaft mit den darin enthaltenen verderblichen Inhaltsstoffen Zucker und Proteine sofort mit dem Pressaft der normalen Sommerproduktion mitverarbeitet wird, während der faserreiche Rückstand unter Zugabe von Milchsäurebakterien einsiliert wird. Dies erlaubt eine kompakte Lagerung in hoher Lagerdichte, was die Lagerverluste minimiert, während die Trocknungskosten entfallen.
- Das erfindungsgemässe verfahrenstechnische Vorgehen bezüglich Pressen und Vorbehandlung des Rohstoffes führt zu einer deutlichen Reduktion der zur Dampfbehandlung benötigten Dampfmenge.
- Die erfindungsgemässe verfahrenstechnische Bearbeitung (Handling) der vorbehandelten Fasern erhöht die Betriebssicherheit und ergibt noch weitere verfahrenstechnische Vorteile.
- Die erfindungsgemässe Verpressung der dampfbehandelten Phase ermöglicht die Rückgewinnung von aus Kondensat unter Überdruck bestehendem Fluid und reduziert den mit der Entspannung verbundenen Energieverlust. Während die Temperatur des Dampfes nach der Entspannung nur 100°C beträgt, beträgt die Temperatur des genannten Fluids vor der Entspannung genau die eingestellten Betriebswerte. Mit Vorteil wird das genannte Fluid zur Vorerwärmung des Rohstoffes genutzt.
- Die abgeschiedenen feststoffarmen Phasen sind, gegebenenfalls vereinigt, nach einer Hygienisierung als Fermentationsmedium nutzbar. Auch kann das darin enthaltene Protein koaguliert, abgetrennt und genutzt werden. Zudem können darin je nach den verwendeten Rohstoffen wertvolle Inhaltstoffe wie Limonene aus Rückständen der Citrusverarbeitung, Tannine aus Mostereirückständen usw. enthalten sein, die abgetrennt und genutzt werden. Diese Weiterverarbeitung und Nutzung trägt zur Optimierung der Wirtschaftlichkeit des erfindungsgemässen Verfahrens erheblich bei.
- In den abgeschiedenen feststoffarmen Phasen unterliegt der Gehalt an fermentierbaren Zuckern je nach Rohstoff und Jahreszeit grossen Schwankungen, die durch Mitverarbeitung von gewaschenen Zuckerrüben ausgeglichen werden können. Auf diese Weise kann der Wert der feststoffarmen Phasen erhöht und deren Nutzung als Fermentationsmedium stabilisiert werden. Dabei kann der Pressrückstand der Zuckerrüben zusammen mit der feststoffreichen Phase weiterverarbeitet werden. Gesamthaft ergibt sich daraus eine Anpassung des Verfahrens an die betreffenden mengen- und wertmässigen saisonalen Schwankungen.

Die erfindungsgemäss bevorzugte Kühlung der dampfbehandelten feststoffreichen Phase durch direkte Kontaktierung mit dem Kühlfluid in zwei aufeinanderfolgenden Kühlstufen vermag die folgenden Aufgaben zu lösen:
- Die Anlage kann kompakter und daher kostengünstiger gebaut werden als mit einer indirekten Kühlung, bei welcher wegen des schlechten Wärmeübergangs eine grosse Wärmeaustauschfläche erforderlich wäre.
- Mit den beiden Kühlstufen und der dazwischen eingesetzten Zwischenstufe wird es möglich, die feststoffreiche Phase auf eine Temperatur zu bringen, welche für die verwendeten Enzyme und Mikroorganismen optimal ist, und letztere genau unter diesen Bedingungen beizugeben, beispielsweise beim bekannten Temperaturoptimum der enzymatischen Verzuckerung bei 40-65°C. Zudem erfolgt die Zugabe von Enzymen mit Vorteil zu frisch vorbehandelten Fasern, statt zum Reaktionsgemisch im Fermenter.
- Mit den beiden Kühlstufen und der dazwischen eingesetzten Zwischenstufe wird es möglich, unterschiedliche Betriebsbedingungen miteinander kompatibel zu machen. Durch entsprechende Dimensionierung und Änderung der Zwischenstufe ist es beispielsweise möglich, eine Vorbehandlung während 8-16 Stunden pro Tag und eine kontinuierliche Weiterverarbeitung zusammenzuschalten.
- Dank dem geschlossenen Kreislauf des Kühlfluids gehen keine Enzyme verloren.

Mit der erfindungsgemässen Schneckenpresse wird unter anderem erreicht, dass die vom zusätzlichen Kammerbereich gebildete Schlaufe mit Gutvorschub, welche die beiden Teilbereiche miteinander verbindet, die unabhängige Einstellung der Aufenthaltsdauer der feststoffreichen Phase unter gegebenenfalls unabhängig wählbaren Druck- und Temperaturbedingungen ermöglicht.

Nachstehend wird die Erfindung anhand eines Ausführungsbeispiels unter Bezugnahme auf die Zeichnung näher erläutert. Es zeigt:

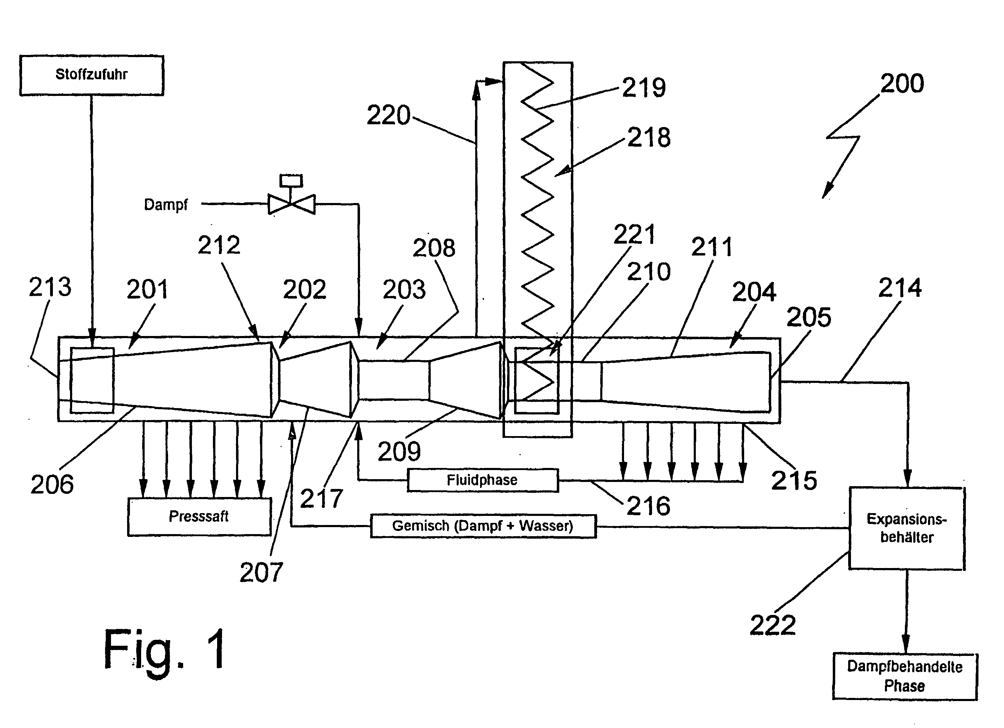

- Fig. 1: eine schematische Darstellung einer Schneckenpresse zur Durchführung des erfindungsgemässen Verfahrens.

Im nachstehenden wird ein Ausführungsbeispiel des erfindungsgemässen Verfahrens beschrieben.

Frisch geschnittenes Klee und Gras mit einem Gehalt an Trockensubstanz von 15-30 Gew.-% und typisch von etwa 20 Gew.-% wird während 8-16 Stunden pro Tag angeliefert und über eine Förder- und Dosierschnecke zu einem Macerator geleitet, der den Rohstoff zu einem Brei verarbeitet.

In einer Schneckenpresse wird von diesem Brei ein Saft abgepresst (Presssaft), der in grösseren Mengen fermentierbare Zucker sowie Rohprotein enthält.

Dieser Saft wird in einem Wärmeaustauscher einer Hitzebehandlung mit Dampf unterzogen, wodurch die Proteine koaguliert werden. Auch wird der Saft dadurch hygienisiert, was ihn als Fermentationsmedium nutzbar macht. Bevorzugte Nutzungen sind die Abtrennung und Aufarbeitung der im Saft enthaltenen Proteine, verbunden mit einer Nutzung der fermentierbaren Zucker als Kohlenstoffquelle für Mikroorganismen. Möglich ist auch eine Überleitung des hygienisierten Saftes in einen oder mehrere mit der feststoffreichen Phase beschickten Fermenter, verbunden mit der späteren Abtrennung und Aufarbeitung der im Saft enthaltenen Proteine zusammen mit der proteinreichen ausfermentierten Fermentationsbrühe.

Die nach der Verpressung bei etwa 30-60°C verbleibende feststoffreiche Phase wird zunächst mit rückgeführtem Fluid (Gemisch von Dampf und Wasser bei etwa 100°C) auf etwa 80°C vorerwärmt, anschliessend in eine Druckkammer weitergeleitet und dort unter Zufuhr von Dampf (vorzugsweise unter 10 bar) und heisser wässriger Phase einer Dampfbehandlung bei etwa 100-170°C und typisch bei etwa 140°C unter etwa 4 bar während etwa 10 Minuten unterzogen.

Von dieser dampfbehandelten Phase wird anschliessend heisse Flüssigkeit unter Druck abgepresst, die generell einer Nutzung zugeführt wird und typisch zum Anfang derselben Dampfbehandlung rückgeführt und dort als heisse wässrige Phase wiederverwendet wird.

Die verbleibende dampfbehandelte feststoffreiche Phase gelangt von der Presse zu einem Expansionsbehälter, wo sie entspannt und auf 100°C gebracht wird. Durch diese Entspannung wird die feststoffreiche Phase gelockert, was deren Förderung und weitere Bearbeitung erleichtert. Das aus der Entspannung resultierende Fluid (Gemisch von Dampf und Wasser bei etwa 100°C) wird bei der vorangehenden Vorerwärmung als rückgeführtes Fluid wiederverwendet.

Die verbleibende dampfbehandelte feststoffreiche Phase wird anschliessend gekühlt. Diese Kühlung erfolgt zweistufig, und zwischen den beiden Kühlstufen ist ein Zwischenbehälter angeordnet. In jeder der beiden Kühlstufen werden das Kühlfluid und die feststoffreiche Phase gemischt, und das resultierende Gemisch wird schliesslich durch Verpressung in Kühlfluid und feststoffreiche Phase getrennt.

In dem Zwischenbehälter werden der feststoffreichen Phase Enzyme, typisch Cellulasen zugeführt, die bei den dort herrschenden etwa 40-70°C und typisch etwa 50°C ihre Wirkung optimal entfalten können. Gleichzeitig dient dieser Zwischenbehälter als Puffer zum Ausgleich zwischen der im vorstehenden beschriebenen, im Rhythmus von 8 bis 16 Stunden pro Tag erfolgenden Vorbehandlung und der darauffolgenden, nun zu beschreibenden kontinuierlichen Weiterverabeitung. Aus dem Zwischenbehälter erfolgt die Überleitung zur zweiten Kühlstufe, in welcher die feststoffreiche Phase auf etwa 30°C gehühlt wird. Von dieser zweiten Kühlstufe erfolgt mittels einer Förderschnecke die Beschickung von Fermentern.

Der Hauptteil der gekühlten Verarbeitungsmenge wird zur gleichzeitigen Verzuckerung und Fermentation in einen oder mehrere Hauptfermenter übergeleitet.

Ein kleinerer Teil der im Zwischenbehälter enthaltenen Substanz, typisch etwa 5-10 Gew.-% der Verarbeitungsmenge, wird in einen separaten Nebenfermenter zur Enzymherstellung geleitet. Dort erfolgt die Enzymherstellung unter den dafür geeigneten Bedingungen, vorzugsweise mit Hilfe von Pilzstämmen der Gattung Trichoderma oder Aspergillus. Die im Nebenfermenter ausfermentierte enzymhaltige Brühe wird dann einschliesslich des darin enthaltenen Mycels in die Hauptfermenter übergeleitet.

In den Hauptfermentern erfolgt, typisch bei 35°C eine Umsetzung der in der feststoffreichen Phase enthaltenen Fasern zu Ethanol. Während dieser Umsetzung wird Kohlendioxid freigesetzt, welches gefasst, verdichtet und in verflüssigter Form zur Vermarktung gelangt.

Die in den Hauptfermentern ausfermentierte Brühe enthält wasserlösliche Stoffe und Feststoffe, die mittels eines Dekanters und/oder weiterer geeigneter Filter abgetrennt werden. Die abgetrennten Feststoffe sind in flüssiger oder getrockneter Form als Tierfutter verwendbar. Die flüssige Phase mit den wasserlöslichen Stoffen wird zur Entnahme von Ethanol in eine Destillationskolonne übergeleitet, die ihren Wärmebedarf von Dampf bezieht.

Nach der Destillation bei etwa 100°C enthält die von Ethanol befreite flüssige Phase vor allem nicht fermentierbare Zucker wie Xylose und Arabinose, und organische Säuren sowie Nährsalze. Die in der flüssigen Phase enthaltene organische Substanz wird in einem Faulbehälter typisch bei etwa 37°C zu Biogas umgesetzt, welches zur Wärmeerzeugung benutzt wird und fast den gesamten Bedarf an Prozesswärme zu decken vermag. Auch bei der entsprechenden Kühlung der organischen Substanz von etwa 100°C auf etwa 37°C wird Warmwasser produziert, das wie vorstehend erwähnt genutzt wird.

Zudem ergibt die Erzeugung von Biogas eine weitgehende Reinigung des Prozesswassers, welches dadurch wiederverwendbar wird.

Auf die vorstehend beschriebene Weise können aus einer Tonne Trockensubstanz des vorstehend erwähnten Rohstoffes etwa 180-250 l Ethanol (mit etwa 95%iger Reinheit), 150-300 kg Proteinkonzentrat (mit etwa 90 Gew.-% Trockensubstanz und etwa 35-50 Gew.-% Rohprotein), 150-300 kg Fasern und 140-200 kg Kohlendioxid hergestellt bzw. gewonnen werden.

Bei der ebenfalls möglichen Herstellung von Backhefe können aus einer Tonne Trockensubstanz des vorstehend erwähnten Rohstoffes etwa 110-180 kg Backhefe (mit etwa 30 Gew.-% Trockensubstanz), 90-160 l Ethanol (mit etwa 95%iger Reinheit), 150-300 kg Proteinkonzentrat (mit etwa 90 Gew.-% Trockensubstanz und etwa 35-50 Gew.-% Rohprotein), 150-300 Fasern und 50-100 kg Kohlendioxid hergestellt bzw. gewonnen werden.

Je nach den Gegebenheiten des Marktes ist es auch möglich, zugunsten einer Maximierung der Backhefe- oder Faserproduktion teilweise oder ganz auf die Ethanolproduktion zu verzichten. Unter solchen Voraussetzungen können pro Tonne Trockensubstanz des vorstehend erwähnten Rohstoffes etwa 180-350 kg Backhefe (mit etwa 30 Gew.-% Trockensubstanz) oder etwa 300-400 kg Fasern hergestellt bzw. gewonnen werden.

Als weitere Optionen ermöglicht das beschriebene Verfahren auch die Herstellung anderer Produkte wie beispielsweise von Citronensäure oder Essigsäure (durch Verwendung der hierfür geeigneten Mikroorganismen), anderen Abbauprodukten (durch Verwendung der hierfür geeigneten Enzyme wie Amylasen, Pektinasen und/oder Hemicellulasen), Xylit und/oder Furfural (durch Weiterverabeitung der in der ausfermentierten Fermentationsbrühe enthaltenen Xylose) usw. Insbesondere kann Zellstoff dadurch gewonnen werden, dass die Dampfbehandlung der nach der Verpressung bei etwa 30-60°C verbleibenden feststoffreichen Phase bei tieferer Temperatur und mit kürzerer Dauer vollzogen wird, als es nötig wäre, um bei der nachfolgenden Einwirkung von Enzymen und/oder Mikroorganismen die bestmögliche Verzuckerung zu erreichen. So bleiben in dieser feststoffreichen Phase nach erfolgtem Abbau wasserunlösliche Polysaccharide erhalten, die einen Zellstoff darstellen.

In der nachstehenden Beschreibung einer Schneckenpresse zur Durchführung des beschriebenen Verfahrens wird auf die in Fig. 1 gegebene schematische Darstellung einer solchen Schneckenpresse Bezug genommen.

Die allgemein mit 200 bezeichnete Schneckenpresse umfasst Kammern 201...204, von denen jede dazu bestimmt ist, auf jeweils zugeführtes Gut Verfahrensschritte der Verpressung und der Behandlung durch eine im wesentlichen feststoffreie wässrige Fluidphase durchzuführen.

Die Kammern 201...204 sind axial aneinandergereiht und mit einer gemeinsamen integralen Welle 205 versehen. Auf dieser Welle 205 sind Schnecken 206...211 für den Gutvorschub in der jeweiligen Kammer angeordnet. Es ist zu verstehen, dass in diesen Kammern auf bekannte Weise auch mehrere integrale Wellen mit jeweiligen Schnecken vorgesehen sein können, beispielsweise jeweils zwei einander parallele, gegeneinander drehende Wellen mit entsprechenden Schnecken.

Beim Betrieb der Schneckenpresse bildet sich am förderseitigen Ende jeder Schnecke (beispielsweise in der Kammer 201 bei 212 am förderseitigen Ende der Schnecke 206) ein Pfropfen des dort verdichteten geförderten Guts. Dieser Pfropfen dichtet die betreffende Kammer (hier beispielsweise 201) von der nachfolgenden Kammer (hier 202) ab, hindert aber nicht das geförderte, zu Pfropfen gepresste Gut dort aus der Kammer herausgepresst zu werden und auf diese Weise von der einen Kammer in die nächste zu gelangen.

Am eingangsseitigen Ende der ersten Kammer 201 wird das zu behandelnde Gut eingeführt, wobei sich dort kein Pfropfen bildet, so dass der Abschluss der Kammer 201 durch eine Wandung 213 zu erfolgen hat.

Aus der letzten Kammer 204 tritt das behandelte Gut aus, indem der Pfropfen in eine Leitung 214 hinausgepresst wird, wie es in der Zeichnung schematisch angedeutet wird.

Die Verpressung des behandelten Gutes in der letzten Kammer 204 ermöglicht, den flüssigen und/oder gasförmigen Teil des behandelten Gutes aus dieser Kammer 204 ohne Temperaturabfall durch Öffnungen 215 zu entnehmen, in einer Leitung 216 zu sammeln, über diese Leitung 216 entlang den Kammern 204...203 zurückzuführen und in die Kammer 203 bei einer Öffnung 217 einzuführen. Dadurch entsteht eine Rückführung der dem behandelten Gut entnommenen wässrigen Fluidphase im wesentlichen entlang einem die Kammern 203...204 umfassenden Kammerbereich in einer dem Gutvorschub entgegengesetzten Richtung.

Dieser die Kammern 203...204 umfassende Kammerbereich ist in zwei axial aneinandergereihte Teilbereiche aufgeteilt, von denen der eine die Kammer 203 mit den Schnecken 208...209 umfasst und der andere die Kammer 204 mit den Schnecken 210...211 umfasst. Alle diese Schnecken sind als Abschnitte der gemeinsamen integralen Welle 205 ausgebildet, und sie bewerkstelligen den Gutvorschub in den betreffenden Kammern.

Auch zwischen den genannten beiden Teilbereichen, also zwischen den Kammern 203 und 204 bildet sich beim Betrieb der Schneckenpresse aus dem dort verdichteten geförderten Gut ein Pfropfen, der die Kammer 203 von der nachfolgenden Kammer 204 abdichtet. An dieser Schnittstelle zwischen den Kammern 203 und 204 ist die Schneckenpresse jedoch so ausgebildet, dass der dort gebildete Pfropfen das geförderte Gut daran hindert, direkt von der Kammer 203 zur Kammer 204 bzw. vom förderseitigen Ende der Schnecke 209 zum aufnahmeseitigen Ende der Schnecke 210 zu gelangen. Hier ist nämlich erwünscht, dass das geförderte Gut zwischen den Kammern 203 und 204 bzw. zwischen den genannten beiden Teilbereichen eine Schlaufe durchläuft, welche die Kammern 203 und 204 bzw. die genannten beiden Teilbereiche miteinander verbindet.

Diese Schlaufe umfasst eine (skizziert dargestellte) Kammer 218 mit einer (skizziert dargestellten) eigenen Schnecke 219 für den Gutvorschub, sowie eine entsprechende (skizziert dargestellte) Zuleitung 220 des zu behandelnden Gutes vom förderseitigen Ende der Schnecke 209 zum aufnahmeseitigen Ende der Kammer 218, und noch eine entsprechende (skizziert als Öffnung dargestellte) Zuleitung 221 des in der Kammer 218 behandelten Gutes vom förderseitigen Ende der Schnecke 219 zum aufnahmeseitigen Ende der Kammerbereiches 204.

An dieser Schlaufe ist die Fördergeschwindigkeit und damit die Aufenthaltsdauer des geförderten Guts unabhängig von den in den Kammern 201...204 bzw. an den Schnecken 206...211 herrschenden Bedingungen einstellbar. Beispielsweise ist auf diese Weise (und mit Hilfe einer entsprechend lang bemessenen Schlaufe) eine längere Aufenthaltsdauer des geförderten Guts auch bei höherer Fördergeschwindigkeit an der Welle 205 erreichbar.

Von der Schneckenpresse 200 gelangt die nun dampfbehandelte feststoffreiche Phase zu einem der Schneckenpresse 200 bzw. der Kammer 204 nachgeschalteten Expansionsbehälter 222, wo sie entspannt, auf 100°C gebracht und dabei auch gelockert wird. Für das aus der Entspannung resultierende Fluid (Gemisch von Dampf und Wasser bei etwa 100°C) ist eine Rückführung zur Kammer 202 der Schneckenpresse 200 im Bereich des förderseitigen Endes der entsprechenden Schnecke 207 vorgesehen und in der Zeichnung skizziert angedeutet.

### Liste der Bezugszeichen

Schneckenpresse 200
Kammern 201...204
Welle 205
Schnecken 206...211
Förderseitiges Ende 212 der Schnecke 206
Wandung 213
Leitung 214
Öffnungen 215
Leitung 216
Öffnung 217
Kammer 218
Schnecke 219
Zuleitung 220
Zuleitung 221
Expansionsbehälter 222

## Patentansprüche

1. Verfahren zur Verwertung von Biomasse aus landwirtschaftlicher Ernte im natürlichen Feuchtigkeitszustand, **gekennzeichnet durch** die Kombination von aufeinanderfolgenden Verfahrensschritten:
(a) Bereitstellung der Biomasse in einer zur Verarbeitung in Schneckenpressen geeigneter zerkleinerter Form;
(b) Verpressung der bereitgestellten Biomasse aus dem Verfahrensschritt (a) unter Trennung der verpressten Biomasse in je eine feststoffarme und eine feststoffreiche Phase, und Abscheidung der feststoffarmen Phase im Hinblick auf deren Verwertung;
(c) Behandlung der feststoffreichen Phase aus dem Verfahrensschritt (b) mit Dampf im Überdruck;
(d) Entspannung des beim Verfahrensschritt (c) aufgebauten Überdrucks unter Trennung der dampfbehandelten Phase aus dem Verfahrensschritt (c) in je eine Dampfphase und eine feststoffreiche Phase, und Abscheidung der Dampfphase im Hinblick auf deren Verwertung;
(e) Kühlung der feststoffreichen Phase aus dem Verfahrensschritt (d) unter Verwertung von dabei rückgewonnener Wärme;
(f) Abbau von Stoffen in der gekühlten Phase aus dem Verfahrensschritt (e) **durch** Einwirkung von Enzymen und/oder **durch** Fermentation mit Mikroorganismen unter Trennung der abbaubehandelten Phase in je eine gasförmige Phase und eine fliessfähige Gemischphase, und Abscheidung der gasförmigen Phase im Hinblick auf deren Verwertung;
(g) Trennung der fliessfähigen Gemischphase aus dem Verfahrensschritt (f) in je eine feststoffarme und eine feststoffreiche Phase, und Abscheidung der feststoffreichen Phase im Hinblick auf deren Verwertung;
(h) Verwertung von feststoffarmer Phase aus mindestens einem der Verfahrensschritte (b) und (g).

2. Verfahren nach Anspruch 1, **dadurch gekennzeichnet, dass** anschliessend an die Dampfbehandlung beim Verfahrensschritt (c) eine Verpressung der dampfbehandelten Phase aus dem Verfahrensschritt (c) unter Trennung der verpressten Phase in je eine feststoffreie wässrige Fluidphase und eine feststoffreiche Phase vorgenommen wird, bevor die Entspannung beim Verfahrensschritt (d) stattfindet, wobei die wässrige Fluidphase abgeschieden und zum Verfahrensschritt (c) rückgeführt wird.

3. Verfahren nach Anspruch 1 oder 2, **dadurch gekennzeichnet, dass** die Kühlung der feststoffreichen Phase beim Verfahrensschritt (e) in zwei Kühlstufen erfolgt, wobei die feststoffreiche Phase zwischen den beiden Kühlstufen eine Zwischenstufe durchläuft und bei diesem Durchlauf zumindest ein Enzym, das beim Verfahrensschritt (f) Stoffe abbaut, der feststoffreichen Phase beigegeben wird.

4. Verfahren nach einem der Ansprüche 1 bis 3, **dadurch gekennzeichnet, dass** der Abbau von Stoffen beim Verfahrensschritt (f) eine alkoholische Gärung umfasst und die beim Verfahrensschritt (g) gewonnene feststoffarme Phase einer Destillation zur Abscheidung von Ethanol unterzogen wird.

5. Verfahren nach einem der Ansprüche 1 bis 3, **dadurch gekennzeichnet, dass** der Abbau von Stoffen beim Verfahrensschritt (f) eine Einwirkung von zuckerproduzierenden Enzymen wie Cellulasen, Amylasen, Pektinasen und/oder Hemicellulasen umfasst.

6. Verfahren nach Anspruch 5, **dadurch gekennzeichnet, dass** die Dampfbehandlung beim Verfahrensschritt (c) bei tieferer Temperatur und/oder mit kürzerer Dauer vollzogen wird, als es für eine bestmögliche Verzuckerung durch Einwirkung von zuckerproduzierenden Enzymen und/oder Mikroorganismen beim Verfahrensschritt (f) erforderlich ist, so dass nach erfolgtem Abbau wasserunlösliche Polysaccharidfasern erhalten bleiben, die von der abbaubehandelten Phase als Zellstoff abgeschieden werden.

7. Verfahren nach einem der Ansprüche 1 bis 6, **dadurch gekennzeichnet, dass** der beim Verfahrensschritt (d) gewonnene Dampf zur Vorerwärmung der beim Verfahrensschritt (c) zu behandelnden feststoffreichen Phase verwendet wird.

8. Verfahren nach einem der Ansprüche 1 bis 7, **dadurch gekennzeichnet, dass** die feststoffarme Phase aus dem Verfahrensschritt (b) und/oder (g) einer anaeroben Faulung unter Trennung der gefäulten Phase in Biogas und einer fliessfähigen Phase unterzogen, das Biogas abgeschieden und zumindest ein Teil davon als Brennstoff verwendet wird.

9. Schneckenpresse zur kontinuierlichen Durchführung des Verfahrens nach Anspruch 2,
**gekennzeichnet durch** Kammerbereiche zur jeweiligen Durchführung von Schritten der Verpressung und der Behandlung **durch** eine feststoffreie wässrige Fluidphase auf jeweils zugeführtem Gut, wobei
die Kammerbereiche (201...204) axial aneinandergereiht, mit mindestens einer gemeinsamen integralen Welle (205) mit daran angeordneten Schnecken (206...211) für den Gutvorschub versehen und beim Betrieb der Schneckenpresse (200) **durch** einen Propfen aus verdichtetem Gut voneinander abgedichtet sind, und
an und entlang einem der Kammerbereiche (203...204) eine Rückführung der wässrigen Fluidphase in einer dem Gutvorschub entgegengesetzten Richtung vorgesehen ist.

10. Schneckenpresse nach Anspruch 9, **dadurch gekennzeichnet, dass**
der mit der Rückführung versehene 'Kammerbereich (203...204) in zwei axial aneinandergereihte Teilbereiche (203, 204) aufgeteilt ist,
jeder Teilbereich (203, 204) mit einer als Abschnitt der gemeinsamen integralen Welle (205) ausgebildeten Schnecke (208...209; 210...211) für den Gutvorschub versehen ist,
die beiden Teilbereiche (203, 204) beim Betrieb der Schneckenpresse (200) durch einen Propfen aus verdichtetem Gut voneinander abgedichtet sind, und
zwischen den beiden Teilbereichen (203, 204) ein mit einer separaten eigenen Schnecke (219) für den Gutvorschub versehener zusätzlicher Kammerbereich (218) vorgesehen ist, der die beiden Teilbereiche (203, 204) miteinander verbindet und dazwischen eine Schlaufe mit Gutvorschub (220...221) bildet.

11. Verwendung des Verfahrens nach einem der Ansprüche 1 bis 8 zur Herstellung von Proteinkonzentrat.

12. Verwendung des Verfahrens nach einem der Ansprüche 1 bis 8 zur Gewinnung von Ethanol sowie zur Herstellung von Proteinkonzentrat und von Fasern.

13. Verwendung des Verfahrens nach einem der Ansprüche 1 bis 8 zur Gewinnung von Ethanol sowie zur Herstellung von Proteinkonzentrat und von Fasern aus einer aus Gras bestehender Biomasse.

## Claims

1. A method of utilizing biomass from agricultural crop in its naturally moist state, **characterized by** the combination of successive method steps:
(a) Providing the biomass in a chopped form appropriate to processing in screw presses;
(b) Pressing the provided biomass from method step (a) and concomitant separation of the pressed biomass into each of a phase of low solids content and a phase of high solids content, and separation of the phase of low solids content for the latter's utilization;
(c) Processing the phase of high solids content from method step (b) with pressurized vapor;
(d) Releasing pressure of the pressurization built-up at method step (c) and concomitant separation of the vapor-treated phase from method step (c) into each of a vapor phase and a phase of high solids content, and separation of the vapor phase for the latter's utilization;
(e) Cooling the phase of high solids content from method step (d) and concomitant utilization of heat recovered in this step;
(f) Decomposition of materials in the cooled phase from method step (d) by the action of enzymes and/or by fermentation with microorganisms and concomitant separation of the decomposed phase into each of a gaseous phase and a phase capable of flowing, and separation of the gaseous phase for the latter's utilization;
(g) Separation of the phase capable of flowing from method step (f) into each of a phase of low solids content and a phase of high solids content, and separation of the phase of high solids content for the latter's utilization;
(h) Utilization of the phase of low solids content from at least one of the method steps (b) and (g).

2. The method according to Claim 1, **characterized in that**, immediately following the vapor treatment of method step (c) there is performed a pressing of the vapor-treated phase from method step (c) and a concomitant separation of the pressed phase into each of an aqueous fluid phase devoid of solids and a phase of high solids content, before the release of pressure in method step (d) takes place, whereby the aqueous fluid phase is separated out and recycled to method step (c).

3. The method according to Claim or 2, **characterized in that** the cooling of the phase of high solids content in method step (e) proceeds in two stages, whereby the phase of high solids content runs through an intermediate stage between the two cooling stages and during this run at least one enzyme that decomposes materials in method step (f) is added to the phase of high solids content.

4. The method according to any of Claims 1 through 3, **characterized in that** the decomposition of materials in method step (f) includes an alcoholic fermentation and the phase of low solids content produced in method step (g) is subjected to a distillation for separation of ethanol.

5. The method according to any of Claims 1 through 3, **characterized in that** the decomposition of materials in method step (f) includes an action of sugar-producing enzymes such as cellulases, amylases, pectinases and/or hemicellulases.

6. The method according to Claim 5, **characterized in that** the vapor treatment in method step (c) is carried out at a lower temperature and/or in a shorter time than would be necessary to obtain the best possible saccharification through the action of sugar-producing enzymes and/or microorganisms, so that after a full decomposition water-insoluble polysaccharides are conserved that are separated out from the decomposed phase as cellulosic material.

7. The method according to any of Claims 1 through 6, **characterized in that** the vapor recovered in method step (d) is utilized for a pre-heating of the phase of high solids content to be processed in method step (c).

8. The method according to any of Claims 1 through 7, **characterized in that** the phase of low solids content from method step (b) and/or (g) is subjected to anaerobic decomposition and concomitant separation of the decomposed phase into fermentation gas and a phase capable of flowing, the fermentation gas is separated and at least part of the latter is utilized as fuel.

9. A screw press for continuously performing the method according to Claim 2,
**characterized by** chamber regions for the respective carrying out of stages of pressing and treating material each time provided thereto with an aqueous fluid phase devoid of solids, whereby
the chamber regions (201...204) are axially disposed inline with at least one common integral shaft (205) having screws (206...211) arranged thereon for conveyance of material and are, during the operation of the screw press (200), sealed off from each other by a plug of compressed material, and
on and along one of the chamber regions (203...204) there is provided a recycling of the aqueous fluid phase in a direction opposite to that of the conveyance of material.

10. A screw press according to Claim 9, **characterized in that**
the chamber region (203...204) provided with the recycling is divided into two sub-regions (203, 204) axially disposed inline next to each other,
each sub-region (203, 204) is provided with a screw (208...209; 210...211) for the conveyance of material that is formed as a section of the common integral shaft (205),
the two sub-regions (203, 204) are sealed off from each other during operation of the screw press (200) by a plug of compressed material, and
between the two sub-regions (203, 204) there is provided an additional chamber region (218) that has its own separate screw (219) for the conveyance of material and that links the two sub-regions (203, 204) with each other and forms in between a material conveyance loop (220...221).

11. Use of the method according to any of Claims 1 through 8 for the production of protein concentrate.

12. Use of the method according to any of Claims 1 through 8 for the recovery of ethanol as well as for the production of protein concentrate and of fibers.

13. Use of the method according to any of Claims 1 through 8 for the recovery of ethanol as well as for the production of protein concentrate and of fibers from a biomass made up of grass.

## Revendications

1. Procédé d'exploitation d'une biomasse provenant de récoltes agricoles à l'état naturel d'humidité, **caractérisé par** la combinaison des étapes suivantes du procédé:
(a) préparation de la biomasse sous une forme fragmentée convenant au traitement dans des presses à vis sans fin ;
(b) pressage de la biomasse préparée provenant de l'étape du procédé (a) en séparant la biomasse pressée en une phase pauvre en matières solides et une phase riche en matières solides, et séparation de la phase pauvre en matières solides en vue de son exploitation;
(c) traitement de la phase riche en matières solides provenant de l'étape du procédé (b) avec de la vapeur sous surpression ;
(d) réduction de la surpression constituée au cours de l'étape du procédé (c) en séparant la phase traitée à la vapeur provenant de l'étape du procédé (c) en une phase de vapeur riche en matières solides, et séparation de la phase de vapeur en vue de son exploitation ;
(e) refroidissement de la phase riche en matières solides provenant de l'étape du procédé (d) avec exploitation de la chaleur ainsi récupérée ;
(f) dégradation de substances dans la phase refroidie provenant de l'étape du procédé (e) par action d'enzymes et/ou par fermentation avec des microorganismes, avec séparation de la phase traitée avec dégradation en une phase gazeuse et une phase mixte coulante, et séparation de la phase gazeuse en vue de son exploitation ;
(g) séparation de la phase mixte coulante provenant de l'étape du procédé (f) en une phase pauvre en matières solides et une phase riche en matières solides, et séparation de la phase riche en matières solides en vue de son exploitation ;
(h) exploitation de la phase pauvre en matières solides provenant d'au moins l'une des étapes du procédé (b) et (g).

2. Procédé selon la revendication 1, **caractérisé en ce qu'**à la suite du traitement à la vapeur de l'étape du procédé (c), il est procédé à un pressage de la phase traitée à la vapeur provenant de l'étape du procédé (c) avec séparation de là phase pressée en une phase fluide aqueuse exempte de matières solides et une phase riche en matières solides, avant qu'ait lieu la détente de l'étape du procédé (d), la phase fluide aqueuse étant séparée et renvoyée à l'étape du procédé (c).

3. Procédé selon la revendication 1 ou 2, **caractérisé en ce que** le refroidissement de la phase riche en matières solides au cours de l'étape du procédé (e) s'effectue en deux étapes de refroidissement, la phase riche en matières solides traversant, entre les deux étapes de refroidissement, une étape intermédiaire et, au cours de ce passage, au moins une enzyme qui dégrade les substances au cours de l'étape du procédé (f) étant ajoutée à la phase riche en matières solides.

4. Procédé selon l'une des revendications 1 à 3, **caractérisé en ce que** la dégradation de substances au cours de l'étape du procédé (f) comprend une fermentation alcoolique et la phase pauvre en matières solides, obtenue au cours de l'étape du procédé (g), étant soumise à une distillation pour séparer l'éthanol.

5. Procédé selon l'une des revendications 1 à 3, **caractérisé en ce que** la dégradation de substances au cours de l'étape du procédé (f) comprend une action d'enzymes produisant du sucre telles que cellulases, amylases, pectinases et/au hémicellulases.

6. Procédé selon la revendication 5, **caractérisé en ce que** le traitement à la vapeur au cours de l'étape du procédé (c) s'effectue à une température plus basse et/ou avec une durée plus courte qu'il n'est nécessaire pour la meilleure saccharification possible par action d'enzymes et/ou de microorganismes produisant du sucre au cours de l'étape du procédé (f), ce qui fait qu'à l'achèvement de la dégradation il reste des fibres de polysaccharide insolubles dans l'eau qui sont séparées de la phase traitée par dégradation, sous forme de cellulose.

7. Procédé selon l'une des revendications 1 à 6, **caractérisé en ce que** la vapeur obtenue au cours de l'étape du procédé (d) est utilisée pour préchauffer la phase riche en substances solides à traiter au cours de l'étape du procédé (c).

8. Procédé selon l'une des revendications 1 à 7, **caractérisé en ce que** la phase pauvre en matières solides provenant de l'étape du procédé (b) et/ou (g) est soumise à une fermentation anaérobie avec séparation de la phase fermentée en biogaz et une phase coulante, le biogaz est séparé et au moins une partie de celui-ci est utilisée comme combustible.

9. Presse à vis sans fin pour la mise en oeuvre continue du procédé selon la revendication 2, **caractérisée par** des zones de chambre pour la réalisation respective d'étapes du pressage et du traitement par une phase fluide aqueuse exempte de matières solides sur le produit alimenté, dans laquelle les zones de chambre (201... 204) sont juxtaposées axialement avec au moins un arbre (205) intégré commun avcc vis sans fin (206... 211) disposées sur celui-ci pour l'avance du produit et sont rendues étanches les unes par rapport aux autres, pendant le fonctionnement de la presse à vis sans fin (200), par un bouchon de produit compacté, et il est prévu, sur et le long de l'une des zones de chambre (203... 204), un retour de la phase fluide aqueuse dans un sens opposé à l'avance du produit.

10. Presse à vis sans fin selon la revendication 9, **caractérisée en ce que** la zone de chambre (203... 204) pourvue du retour est divisée en deux zones partielles (203, 204) juxtaposées axialement, chaque zone partielle (203, 204) est pourvue d'une vis sans fin (208... 209 ; 210... 211), réalisée comme tronçon de l'arbre (209) intégré commun, pour l'avance du produit,
les deux zones partielles (203, 204) sont rendues étanches l'une par rapport à l'autre, pendant le fonctionnement de la presse à vis sans fin (200), par un bouchon de produit compacté, et
entre les deux zones partielles (203, 204) est prévue une zone de chambre (218) supplémentaire, pourvue de sa propre vis sans fin (209) séparée pour l'avance du produit, laquelle zone de chambre relie entre elles les deux zones partielles (203, 204) et forme entre elles une boucle avec avance du produit (220... 221).

11. Utilisation du procédé selon l'une des revendications 1 à 8, pour produire un concentré de protéine.

12. Utilisation du procédé selon l'une des revendications 1 à 8, pour obtenir de l'éthanol ainsi que pour produire un concentré de protéine et des fibres.

13. Utilisation du procédé selon l'une des revendications 1 à 8, pour obtenir de l'éthanol ainsi que pour produire un concentré de protéine et des fibres à partir d'une biomasse constituée d'herbes.
